(19) Europäisches Patentamt
European Patent Office
Office européen des brevets

(11) EP 0 900 212 B1

(12) EUROPÄISCHE PATENTSCHRIFT

(45) Veröffentlichungstag und Bekanntmachung des Hinweises auf die Patenterteilung:
**17.12.2003 Patentblatt 2003/51**

(21) Anmeldenummer: **97923757.5**

(22) Anmeldetag: **22.04.1997**

(51) Int Cl.[7]: **C07D 307/58**, A61K 31/34

(86) Internationale Anmeldenummer:
**PCT/DE97/00826**

(87) Internationale Veröffentlichungsnummer:
**WO 9704/0032 (30.10.1997 Gazette 1997/46)**

# (54) CHIRALE PHENYLDIHYDROFURANONE ALS PDE-IV INHIBITOREN

CHIRAL PHENYLDIHYDROFURANONES AS PDE-IV INHIBITORS

PHENYLDIHYDROFURANONES CHIRALES SERVANT D'INHIBITEURS DE LA PDE-IV

(84) Benannte Vertragsstaaten:
**AT BE CH DE DK ES FI FR GB GR IE IT LI LU MC NL PT SE**

(30) Priorität: **23.04.1996 DE 19617864**

(43) Veröffentlichungstag der Anmeldung:
**10.03.1999 Patentblatt 1999/10**

(73) Patentinhaber: **Schering AG**
**13353 Berlin (DE)**

(72) Erfinder:
- **LAURENT, Henry**
  **D-13467 Berlin (DE)**
- **ESPERLING, Peter**
  **D-12107 Berlin (DE)**
- **HAMP, Kurt**
  **D-13599 Berlin (DE)**
- **SCHNEIDER, Herbert**
  **D-10707 Berlin (DE)**
- **WACHTEL, Helmut**
  **D-14057 Berlin (DE)**

(56) Entgegenhaltungen:
**WO-A-87/06576** **WO-A-92/07567**
**WO-A-94/06423** **DE-A- 2 655 369**
**DE-A- 2 745 320**

- **DRUG DEVELOPMENT RES., Bd. 21, Nr. 2, 1990, Seiten 135-142, XP002039409 KOE,B.K. ET AL.: "Effects of Novel Catechol Ether Imidazolidinones on Ca-Independent PD Activity, [3H]Rolipram Binding, and Reserpine-Induced Hypothermia in Mice"**
- **J.MED.CHEM., Bd. 32, Nr. 7, 1989, WASHINGTON, Seiten 1450-1457, XP002039410 MARIVET,M.C. ET AL.: "Inhibition of Cyclic Adenosine-3,5-monophosphate Phosphodiesterase from Vascular Smooth Muscle by Rolipram Analogues "**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue chirale Phenyldihydrofuranon-Derivate, das Verfahren zu deren Herstellung und deren Verwendung als Arzneimittel.

Es ist bekannt, daß selektive Inhibitoren der cAMP-spezifischen Phosphodiesterase IV (cAMP-PDE IV-Inhibitor) pharmakologische Eigenschaften besitzen, auf Grund derer sie geeignete Wirkstoffe in Arzneimittelzubereitungen darstellen. Um die Wirksamkeit zu verbessern, wurden zahlreiche Modifizierungen am Molekül vorgenommen, ohne jedoch die gewünschte Wirkungssteigerung zu erzielen. Stickstoff-enthaltende Heterocyclen sind aus DE-A-2655369, DE-A-2745320, WO-A-87/06576, WO-A-92/07567, WO-A-94/06423, Drug Develop. Res. 21, 125 (1990) und J. Med. Chem. 32, 1450 (1989) bekannt.

Überraschenderweise wurde nun gefunden, daß die Verbindungen der Formel I sehr potente selektive cAMP-PDE IV-Inhibitoren darstellen, die metabolisch sehr stabil sind.

Die Erfindung betrifft racemische und enantiomerenreine Verbindungen der Formel I,

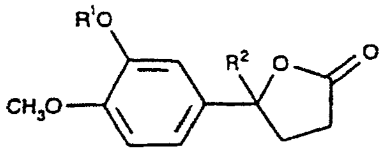

(I)

worin

$R^1$ einen Kohlenwasserstoffrest mit bis zu 8 C-Atomen und

$R^2$ $C_{1-4}$-Alkyl bedeutet.

Als Kohlenwasserstoffrest $R^1$ ist Alkyl, Alkenyl, Alkinyl, Cycloalkyl und Cycloalkylalkyl geeignet.

Der Alkylrest $R^1$ hat vorzugsweise bis zu 6 Kohlenstoffatome und kann wie der Alkylrest $R^2$ geradkettig oder verzweigt sein wie beispielsweise Methyl, Ethyl, Propyl, Isopropyl, Butyl, *sek*-Butyl, Isobutyl, *tert*-Butyl, Pentyl, 2-Methyl-butyl, 2,2-Dimethylpropyl und Hexyl.

Bedeutet der Kohlenwassertoffrest Alkenyl oder Alkinyl, so sei beispielsweise Vinyl, 1-Propenyl, 2-Propenyl, 3-Methyl-2-propenyl und 2-Propinyl mit bis zu 4 C-Atomen genannt.

Der Cycloalkylrest hat 3-7 C-Atome wie beispielsweise Cyclopropyl, Cyclobutyl, Cyclopentyl, Cyclohexyl oder Cycloheptyl.

Als Cycloalkylalkylrest $R^2$ kommt $C_{3-7}$-Cycloalkyl-$C_{1-2}$-alkyl in Betracht wie beispielsweise Cyclopropylmethyl, Cyclopropylethyl, Cyclopentylmethyl u. a. in Betracht.

Die Verbindungen der Formel I enthalten ein oder mehrere chirale Zentren und umfassen die razemisch diastereomeren Gemische sowie die einzelnen Isomeren.

Gegenstand der Erfindung ist auch das Verfahren zur Herstellung der Verbindungen der Formel I und deren Isomeren.

Die Herstellung der Verbindungen der Formel I ist dadurch gekennzeichnet, daß man eine Verbindung der Formel II

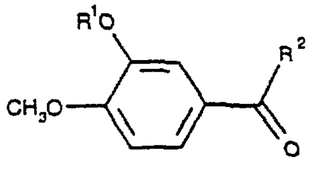

(II)

worin $R^1$ und $R^2$ die obige Bedeutung haben, mit einem Acrylsäureester in Gegenwart von Samarium(II)-iodid und einem Protonendonator umsetzt und gewünschtenfalls anschliessend die Isomeren trennt.

Die durch Samarium(II)-iodid induzierte Kupplung des Acrylsäureesters und einer Verbindung der Formel II wird im allgemeinen bei Raumtemperatur vorgenommen, indem man 2 Molequivalent Samarium(II)-iodid und und 1 Molequivalent Substrat in einem Lösungsmittel umsetzt. Als Lösungsmittel ist insbesondere Tetrahydrofuran geeignet. Samarium(II)-iodid in 0.1 molarer Lösung in THF, stabilisiert mit Samariumpulver, kann von der Firma ALDRICH bezogen werden. Der Zusatz eines Alkohols als Protonendonator wie beispielsweise Methanol, Ethanol oder *tert*-Butanol verhindert die Entstehung von Nebenprodukten und ermöglicht die Herstellung der erfindungsgemäßen Verbindungen unter milden Bedingungen in sehr guter Ausbeute. Die Umsetzung kann nach wenigen Minuten oder auch mehreren Stunden beendet sein.

Durch Zusatz eines Additivs, wie Tetramethylethylendiamin oder Hexamethylphosphoramid, kann die Reaktionsgeschwindigkeit erhöht und die Ausbeute gesteigert werden. Als Ester der Acrylsäure sind insbesondere Alkylester geeignet, jedoch sind auch andere Ester einsetzbar.

Die Isomeren können nach den üblichen Methoden, wie beispielsweise Chromatographie, Kristallisation oder Überführung in Diastereomerengemische mit chiralen Hilfsstoffen, in die Enantiomeren getrennt werden. Die optisch aktiven Phenyldihydrofuranone der Formel I können aus den entsprechenden Racematen durch Chromatographie an chiralen Säulen erhalten werden.

Die neuen Verbindungen der Formel I und deren Isomere sind Inhibitoren der Phosphodiesterase IV und beeinflussen über den gezielten Eingriff in den cAMP-Metabolismus die Signalfunktion dieses bedeutenden intrazellulären Transmitters, welcher das molekulare Substrat des Informationstransfers bei einer Vielfalt biochemischer Reaktionen darstellt. Die verschiedenen Teilaspekte des Wirkprofils lassen sich auf diesen pharmakologischen Basismechanismus zurückführen, welcher eine Erklärungsgrundlage liefert u. a. für den antiinflammatorischen Effekt und die gute neuropsychotrope Wirksamkeit.

Auf Grund ihres Wirkprofils eignen sich die Verbindungen der Formel I zur Behandlung neurodegenerativer Erkrankungen wie Demenz, Morbus Parkinson, Depression, inflammatorischer Erkrankungen wie Arthritis, Schock, Sepsis, pulmonare Erkrankungen, Knochenresorptionserkrankungen, HIV-Infektion, cerebrale Malaria, Multiple Sklerose und inflammatorischer Hauterkrankungen. Ferner zeigen die erfindungsgemäßen Verbindungen auch bronchospasmolytische, antiproliferative, thrombozytenaggregationshemmende und tokolytische Eigenschaften.

Die Verbindungen der Formel I inhibieren auch die TNF-Produktion und sind daher zur Behandlung von Krankheiten geeignet, die über die Aktivierung von TNF vermittelt werden.

Unter Krankheiten, die durch TNF vermittelt werden, sind sowohl Krankheiten zu verstehen, die durch Produktion von TNF ausgelöst werden wie auch Krankheiten, bei denen durch TNF andere Cytokine wie beispielsweise Il-1 oder Il-6 beeinflußt werden.

Unter TNF ist sowohl TNF-$\alpha$ als auch TNF-$\beta$ zu verstehen, die beide durch die Verbindungen der Formel I antagonisiert werden. Bevorzugt wird TNF-$\alpha$ inhibiert.

Die Verbindungen der Formel I eignen sich daher zur Herstellung eines pharmazeutischen Präparates, das zur Behandlung und Prophylaxe von Erkrankungen in Lebewesen verwendet wird, die durch Stimulation von TNF ausgelöst werden. Als Erkrankungen, die durch excessive oder unregulierte TNF-Stimulation beeinflußt werden sind beispielsweise allergische und inflammatorische Erkrankungen, Autoimmunerkrankungen, pulmonare Erkrankungen, infektiöse Erkrankungen und Knochenresorptionserkrankungen bekannt wie Rheumatoide Arthritis, Rheumatoide Spondylitis, Osteoarthritis, Gicht, Sepsis, Septischer Schock, Endotoxin-Schock, Gramnegative Sepsis, Toxisches Schocksyndrom, ARDS (Akutes Atemnotsyndrom), Pulmonare Sarkoidose, Asthma, chronische Bronchitis, Allergische Rhinitis, Konjunktivitis, Fibrose einschließlich cystischer Fibrose und Atherosklerose, Pyresis, Schmerzen, Glomerulonephritis, Silikose, Kachexie, Colitis ulcerosa, Colitis granulomatosa, Morbus Crohn, Osteoporose, Organschädigung nach Reperfusion.

Ferner sind die Verbindungen geeignet zur Behandlung infektiöser Erkrankungen wie viralen Infektionen mit zum Beispiel HIV-, Boma-, Epstein-, Barr-, Masern-, Arboviren-Erregern speziell des zentralen Nervensystems, parasitären Infektionen wie z.B. cerebraler Malaria, Fieber, Myalgie, HIV, AIDS, Cachexie und Rinderwahnsinn. Andere Erkrankungen, die mit den Verbindungen der Formel I behandelt werden können, sind Multiple Sklerose, Lepra, Diabetes mellitus, Diabetes insipidus, Leukämie, Panencephalitis, cerebrale Störungen wie Depression, Senile Demenz, Multiinfarkt, Demenz und Schlaganfall. Die Verbindungen der Formel I können auch zur Behandlung inflammatorischer Hauterkrankungen eingesetzt werden wie beispielsweise Psoriasis, Urticaria, Atopische Dermatitis, Allergische Rhinitis, Kontaktdermatitis, Rheumatische Arthritis, Lupus Erythematosus, Sonnenbrand, Ekzem.

Die Wirksamkeit der Verbindungen der Formel I in den genannten Indikationen kann durch entsprechende übliche pharmakologische Teste gezeigt werden wie anhand der für Phosphodiesterase Typ IV (PDE IV)-Inhibitoren charakteristischen Head twitch- und Grooming-Reaktion an Ratten. Männlichen Wistar-Ratten wird die Verbindung intraperitoneal (i. p.) verabreicht und das Auftreten von Head twitches und Grooming von der 15. bis 75. Minute nach Injektion durch Beobachtung erfaßt.

Die Wirkung auf das Zentralnervensystem wird durch Messung des Verdrängungsvermögens von radioaktiv markiertem Rolipram von Rattenhirnhomogenaten in vitro bestimmt (Eur. J. Pharmacol., Vol. 127, 105-115 (1986)). Die $IC_{50}$-Werte (diejenige Konzentration, bei der 50 % Hemmwirkung eintritt) wurden in die Inhibitionskonstante $K_i$ umgewandelt, die nach folgender Formel berechnet wird:

$$K_i = IC_{50} / [1 + (L/K_D)],$$

worin L die Konzentration des radioaktiven Tracers und $K_D$ die Dissoziationskonstante der $^3$H-Roliprambindung, die getrennt ermittelt wird, bedeutet.

**Tabelle**

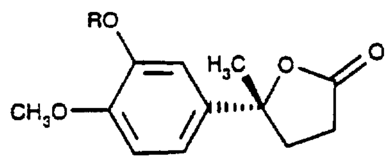

*(R)*-(+)-Isomer

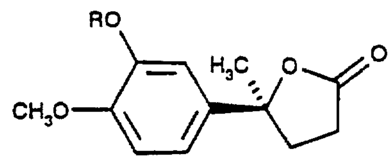

*(S)*-(–)-Isomer

| R | **Racemat** $K_i$ [nM] | ***(R)*-(+)-Enantiomer** $K_i$ [nM] | ***(S)*-(–)-Enantiomer** $K_i$ [nM] |
|---|---|---|---|
| Propyl | 0.74 | 7.30 | 0.47 |
| Cyclopropylmethyl | 0.21 | 2.20 | 0.13 |
| Isobutyl | 0.48 | 2.30 | 0.25 |
| Cyclobutyl | 0.38 | 1.90 | 0.20 |
| Cyclopentyl | 0.50 | 2.80 | 0.28 |

Die Mittel werden nach üblichen Verfahren hergestellt, indem man den Wirkstoff mit geeigneten Träger-, Hilfs- und/oder Zusatzstoffen in die Form eines pharmazeutischen Präparates bringt, das für die enterale oder parenterale Applikation geeignet ist. Die so erhaltenen Zubereitungen können als Arzneimittel in der Human- oder Veterinärmedizin eingesetzt werden. Die Applikation kann oral oder sublingual als Feststoff in Form von Kapseln oder Tabletten oder als Flüssigkeit in Form von Lösungen, Suspensionen, Elexieren, Aerosolen oder Emulsionen oder rektal in Form von Suppositorien oder in Form von gegebenenfalls auch subcutan, intramuskulär oder intravenös anwendbaren Injektionslösungen oder topisch oder intrathekal erfolgen. Als Hilfsstoffe für die gewünschte Arzneimittelformulierung sind die dem Fachmann bekannten inerten organischen und anorganischen Trägermaterialien geeignet wie z. B. Wasser, Gelantine, Gummmi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form, z. B. als Tabletten, Dragees, Suppositoren, Kapseln oder in flüssiger Form, z. B. als Lösungen, Suspensionen oder Emulsionen vorliegen.

Als Trägersysteme können auch grenzflächennahe Hilfsstoffe wie Salze, Gallensäuren oder tierische oder pflanzliche Phospholipide und deren Mischungen sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie z. B. Lactose, Mais- oder Kartoffelstärke, geeignet. Die Anwendung kann auch in flüssiger Form erfolgen, wie z. B. als Saft, dem gegebenenfalls Süßstoff beigefügt wird.

Die Verbindungen der Formel I werden in Dosierungen angewendet, die ausreichend sind, die TNF-Produktion auf normale oder geringere Werte herabzusetzen.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0.1-25 mg, bevorzugt 0.5-5 mg, wobei die Dosis als einmal zu verabreichende Einzeldosis oder unterteilt in zwei oder mehreren Tagesdosen gegeben werden kann.

Soweit die Herstellung der Ausgangsverbindungen nicht beschrieben wird, sind diese aus der Literatur bekannt oder analog zu bekannten Verbindungen oder hier beschriebenen Verfahren herstellbar.

Die nachfolgenden Beispiele sollen das erfindungsgemäße Verfahren erläutern.

**Ausgangsverbindungen:**

**3-Cyclopentyloxy-4-methoxyacetophenon**

Eine Lösung von 2.0 g 3-Hydroxy-4-methoxyacetophenon in 60 ml Ethanol wird nach Zugabe von 3.0 g Kaliumcarbonat unter Rühren tropfenweise mit 2.0 ml Bromcyclopentan versetzt und anschließend 5 Stunden unter Rückfluß zum Sieden erhitzt. Nach dem Abfiltrieren der Salze wird das Lösemittel im Vakuum verdampft und der Rückstand bei 170 °C und 0.15 mbar zweimal am Kugelrohr destilliert. Ausbeute 2.4 g, Schmelzpunkt 56 °C.

**3-Cyclobutyloxy-4-methoxyacetophenon**

Eine Lösung von 3.0 g 3-Hydroxy-4-methoxyacetophenon in 60 ml wasserfreiem Dimethylformamid wird unter Stickstoff mit 1.31 g einer 55proz. Natriumhydrid-Ölsuspension versetzt und 30 Minuten bei 60 °C gerührt. Nach Zugabe von 3.0 g Bromcyclobutan wird das Reaktionsgemisch 3 Stunden auf 110 °C erhitzt, anschließend im Vakuum auf die Hälfte eingeengt und in NaCl-haltiges Eiswasser eingerührt. Man extrahiert mit Diethylether, wäscht den Extrakt mit Wasser, trocknet über Natrumsulfat und engt im Vakkum ein. Der ölige Rückstand wird an einer Silicagelsäule (Kromasil 100/10 µm) mit einem Hexan-*tert*-Butylmethylether-Gemisch (4:1) chromatographiert. Man eluiert 1.77 g, Schmelzpunkt 72 °C.

**3-Cyclopentyloxy-4-methoxy-propiophenon**

Eine Suspension von 1.1 g Magnesium in 5 ml Diethylether wird tropfenweise mit einer Lösung von 3 ml Ethyliodid in 11.5 ml Diethlether versetzt. Es bildet sich das entsprechende Grignard-Reagenz. In diese Lösung tropft man innerhalb einer Stunde eine Lösung von 6.61 g 3-Cyclopentyloxy-4-methoxy-benzaldehyd in 65 ml Diethylether. Anschließend wird das Reaktionsgemisch mit 10 ml gesättigter Ammoniumchloridlösung versetzt, der dabei entstandene Niederschlag wird abgesaugt und das Filtrat, die Etherphase, über Natriumsulfat getrocknet und im Vakuum eingedampft. Der Rückstand, bestehend aus 6.5 g rohem 1-(3-Cyclopentyloxy-4-methoxyphenyl)-propanol, wird in 45 ml reinem Aceton gelöst und unter Eiskühlung tropfenweise mit 7 ml 8 N Jones-Reagenz versetzt .Man rührt 15 Minuten bei Raumtemperatur, versetzt mit Wasser und extrahiert mit Ethylacetat. Der Extrakt wird getrocknet und im Vakuum eingedampft. Der ölige Rückstand wird an einer Silicagelsäule (Kromasil 100/10 µm) mit einem Hexan-*tert*-Butylmethylether-Gemisch (4:1) chromatographiert. Man eluiert 5.23 g 3-Cyclopentyloxy-4-methoxy-propiophenon als öliges Produkt.

**Beispiel 1**

**5-(3-Cyclopentyloxy-4-methoxyphenyl)-5-methyl-dihydro-2-furanon**

**A.** 100 ml einer 0.1 M Lösung von Samarium(II)-iodid in Tetrahydrofuran werden unter striktem Sauerstoffauschluß mit der Lösung von 1.0 g 3-Cyclopentyloxy-4-methoxyacetophenon, 500 mg Acrylsäureethylester und 350 mg *tert*-Butanol in 10 ml Tetrahydrofuran versetzt. Die Reaktionslösung wird gut durchmischt, 15 Stunden bei Raumtemperatur stehen gelassen und anschließend in 100 ml 20proz. Chlorwasserstoffsäure eingegossen. Man rührt zwei Stunden bei Raumtemperatur und extrahiert mit Diethylether. Die Etherphase wird mit Natriumthiosulfatlösung und Wasser gewaschen, über Natriumsulfat getrocknet und im Vakuum eingedampft. Der Rückstand, ein gelbes Öl, wird an einer Silicagelsäule (Kromasil 100/10 µm) mit einem Hexan-*tert*-Butylmethylether-Gemisch (4:1) chromatographiert. Es werden 805 mg kristallines *(R,S)*-5-(3-Cyclopentyloxy-4-methoxyphenyl)-5-methyl-dihydro-2-furanon eluiert, Schmelzpunkt 59.6 °C.

**B.** 500 mg *(R,S)*-5-(3-Cyclopentyloxy-4-methoxyphenyl)-5-methyl-dihydro-2-furanon werden an einer ChiraSpher®-Phase (25 µm) mit einem Hexan-Dioxan-Gemisch (15:1) chromatographiert. Es werden 170 mg *(R)*-(+)-5-(3-Cyclopentyloxy-4-methoxyphenyl)-5-methyl-dihydro-2-furanon, ölig, $[\alpha]_D$ = +24.5° ($CHCl_3$), sowie 220 mg *(S)* -(-)-5-(3-Cyclopentyloxy-4-methoxyphenyl)-5-methyl-dihydro-2-furanon, ölig, $[\alpha]_D$ = -24.2° ($CHCl_3$), eluiert.

**Beispiel 2**

**5-(4-Methoxy-3-propoxyphenyl)-5-methyl-dihydro-2-furanon**

**A.** 100 ml einer 0.1 M Lösung von Samarium(II)-iodid in Tetrahydrofuran werden unter striktem Sauerstoffauschluß mit der Lösung von 860 mg 4-Methoxy-3-propoxyacetophenon, 500 mg Acrylsäuremethylester und 350 mg *tert*-Butanol in 5 ml Tetrahydrofuran versetzt. Die Reaktionslösung wird gut durchmischt, zwei Stunden bei Raumtemperatur stehen gelassen und anschließend in 100 ml 20proz. Chlorwasserstoffsäure eingegossen. Man rührt zwei Stunden bei Raumtemperatur und extrahiert mit Diethylether. Die Etherphase wird mit Natriumthiosulfatlösung und Wasser gewaschen, über Natriumsulfat getrocknet und im Vakuum eingedampft. Der ölige Rückstand wird an einer Silicagelsäule (Kromasil 100/10 µm) mit einem Hexan-*tert*-Butylmethylether-Gemisch (4:1) chromatographiert. Es werden 770 mg *(R,S)*-5-(4-Methoxy-3-propoxyphenyl)-5-methyl-dihydro-2-furanon eluiert, Schmelzpunkt 67.0 °C.

**B.** 610 mg *(R,S)*-5-(4-Methoxy-3-propoxyphenyl)-5-methyl-dihydro-2-furanon werden an einer ChiraSpher®-Phase (25 µm) mit einem Hexan-Dioxan-Gemisch (15:1) chromatographiert. Es werden 242 mg *(R)*-(+)-5-(4-Methoxy-3-propoxyphenyl)-5-methyl-dihydro-2-furanon, Schmelzpunkt 82.3 °C, $[\alpha]_D$ = +27.4° ($CHCl_3$), sowie 190 mg *(S)*-(-)-5-(4-Methoxy-3-propoxyphenyl)-5-methyl-dihydro-2-furanon, Schmelzpunkt 83.3 °C, $[\alpha]_D$ = -24.2° ($CHCl_3$), eluiert.

**Beispiel 3**

**5-(3-Isobutyloxy-4-methoxyphenyl)-5-methyl-dihydro-2-furanon**

**A.** 100 ml einer 0.1 M Lösung von Samarium(II)-iodid in Tetrahydrofuran werden unter striktem Sauerstoffauschluß mit der Lösung von 1.35 g 3-Isobutyloxy-4-methoxyacetophenon, 850 mg Acrylsäureethylester und 595 mg *tert*-Butanol in 10 ml Tetrahydrofuran versetzt. Die Reaktionslösung wird gut durchmischt, 3 Stunden bei Raumtemperatur stehen gelassen und anschließend in 100 ml 20proz. Chlorwasserstoffsäure eingegossen. Man rührt zwei Stunden bei Raumtemperatur und extrahiert mit Diethylether. Die Etherphase wird mit Natriumthiosulfatlösung und Wasser gewaschen, über Natriumsulfat getrocknet und im Vakuum eingedampft. Der ölige Rückstand wird an einer Silicagelsäule (Kromasil 100/10 µm) mit einem Hexan-*tert*-Butylmethylether-Gemisch (4:1) chromatographiert. Es werden 890 mg *(R,S)*-5-(3-Isobutyloxy-4-methoxyphenyl)-5-methyl-dihydro-2-furanon als öliges Produkt eluiert

**B.** 500 mg *(R,S)*-5-(3-Isobutyloxy-4-methoxyphenyl)-5-methyl-dihydro-2-furanon werden an einer ChiraSpher®-Phase (25 µm) mit einem Hexan-Dioxan-Gemisch (15:1) chromatographiert. Es werden 190 mg *(R)*-(+)-5-(3-Isobutyloxy-4-methoxyphenyl)-5-methyl-dihydro-2-furanon, ölig, $[\alpha]_D$ = +23.1° ($CHCl_3$), sowie 180 mg *(S)*-(-)-5-(3-Isobutyloxy-4-methoxyphenyl)-5-methyl-dihydro-2-furanon, ölig, $[\alpha]_D$ = -24.0° ($CHCl_3$), eluiert.

**Beispiel 4**

**5-(3-Cyclobutytoxy-4-methoxyphenyl)-5-methyl-dihydro-2-furanon**

**A.** 100 ml einer 0.1 M Lösung von Samarium(II)-iodid in Tetrahydrofuran werden unter striktem Sauerstoffauschluß mit der Lösung von 1.30 g 3-Cyclobutyloxy-4-methoxyacetophenon, 890 mg Acrylsäuremethylester und 625 mg *tert*-Butanol in 10 ml Tetrahydrofuran versetzt. Die Reaktionslösung wird gut durchmischt, 16 Stunden bei Raumtemperatur stehen gelassen und anschließend in 100 ml 20proz. Chlorwasserstoffsäure eingegossen. Man rührt zwei Stunden bei Raumtemperatur und extrahiert mit Diethylether. Die Etherphase wird mit Natriumthiosulfatlösung und Wasser gewaschen, über Natriumsulfat getrocknet und im Vakuum eingedampft. Der ölige Rückstand wird an einer Silicagelsäule (Kromasil 100/10 µm) mit einem Hexan-*tert*-Butylmethylether-Gemisch (4:1) chromatographiert. Es werden 1.0 g *(R,S)*-5-(3-Cyclobutyloxy-4-methoxyphenyl)-5-methyl-dihydro-2-furanon eluiert, Schmelzpunkt 67.6 °C.

**B.** 600 mg *(R,S)*-5-(3-Cyclobutyloxy-4-methoxyphenyl)-5-methyl-dihydro-2-furanon werden an einer ChiraSpher®-Phase (25 µm) mit einem Hexan-Dioxan-Gemisch (15:1) chromatographiert. Es werden 310 mg (*R*)-(+)-5-(3-Cyclobutyloxy-4-methoxyphenyl)-5-methyl-dihydro-2-furanon, Schmelzpunkt 75.7 °C, $[\alpha]_D$ = +30.3° ($CHCl_3$), sowie 210 mg (*S*)-(-)-5-(3-Cyclobutyloxy-4-methoxyphenyl)-5-methyl-dihydro-2-furanon, Schmelzpunkt 75.7 °C, $[\alpha]_D$ = -28.6° ($CHCl_3$), eluiert.

**Beispiel 5**

**5-(3-Cyclopropylmethoxy-4-methoxyphenyl)-5-methyl-dihydro-2-furanon**

**A.** 100 ml einer 0.1 M Lösung von Samarium(II)-iodid in Tetrahydrofuran werden unter striktem Sauerstoffauschluß mit der Lösung von 1.06 g 3-Cyclopropylmethoxy-4-methoxyacetophenon, 890 mg Acrylsäuremethylester und 625 mg *tert*-Butanol in 15 ml Tetrahydrofuran versetzt. Die Reaktionslösung wird gut durchmischt, eine Stunde bei Raumtemperatur stehen gelassen und anschließend in 100 ml 20proz. Chlorwasserstoffsäure eingegossen. Man rührt zwei Stunden bei Raumtemperatur und extrahiert mit Diethylether. Die Etherphase wird mit Natriumthiosulfatlösung und Wasser gewaschen, über Natriumsulfat getrocknet und im Vakuum eingedampft. Der ölige Rückstand wird an einer Silicagelsäule (Kromasil 100/10 µm) mit einem Hexan-*tert*-Butylmethylether-Gemisch (4:1) chromatographiert. Es werden 840 mg *(R,S)*-5-(3-Cyclopropylmethoxy-4-methoxyphenyl)-5-methyl-dihydro-2-furanon eluiert, Schmelzpunkt 88.2 °C.

**B.** 600 mg *(R,S)*-5-(3-Cyclopropylmethoxy-4-methoxyphenyl)-5-methyl-dihydro-2-furanon werden an einer ChiraSpher®-Phase (25 µm) mit einem Hexan-Dioxan-Gemisch (15:1) chromatographiert. Es werden 310 mg *(R)*-(+)-5-(3-Cyclopropylmethoxy-4-methoxyphenyl)-5-methyl-dihydro-2-furanon, Schmelzpunkt 110.4 °C, $[\alpha]_D$ = +23.0° ($CHCl_3$), sowie 270 mg *(S)*-(-)-5-(3-Cyclopropylmethoxy-4-methoxyphenyl)-5-methyldihydro-2-furanon, Schmelzpunkt 109.4 °C, $[\alpha]_D$ = -24.5° ($CHCl_3$), eluiert.

**Beispiel 6**

**5-(3,4-Dimethoxyphenyl)-5-methyl-dihydro-2-furanon**

100 ml einer 0.1 M Lösung von Samarium(II)-iodid in Tetrahydrofuran werden unter striktem Sauerstoffauschluß mit der Lösung von 710 mg 3,4-Dimethoxyacetophenon, 500 mg Acrylsäuremethylester und 350 mg *tert*-Butanol in 5 ml Tetrahydrofuran versetzt. Die Reaktionslösung wird gut durchmischt, zwei Stunden bei Raumtemperatur stehen gelassen und anschließend in 100 ml 20proz. Chlorwasserstoffsäure eingegossen. Man rührt zwei Stunden bei Raumtemperatur und extrahiert mit Diethylether. Die Etherphase wird mit Natriumthiosulfatlösung und Wasser gewaschen, über Natriumsulfat getrocknet und im Vakuum eingedampft. Der ölige Rückstand wird an einer Silicagelsäule (Kromasil 100/10 µm) mit einem Hexan-*tert*-Butylmethylether-Gemisch (4:1) chromatographiert. Es werden 570 mg *(R,S)*-5-(3,4-Dimethoxyphenyl)-5-methyl-dihydro-2-furanon eluiert, Schmelzpunkt 71:2 °C.

**Beispiel 7**

**5-(3-Ethoxy-4-methoxyphenyl)-5-methyl-dihydro-2-furanon**

100 ml einer 0.1 M Lösung von Samarium(II)-iodid in Tetrahydrofuran werden unter striktem Sauerstoffauschluß mit der Lösung von 1.12 g 3-Ethoxy-4-methoxyacetophenon, 890 mg Acrylsäuremethylester und 625 mg *tert*-Butanol in 8 ml Tetrahydrofuran versetzt. Die Reaktionslösung wird gut durchmischt, 15 Stunden bei Raumtemperatur stehen gelassen und anschließend in 100 ml 20proz. Chlorwasserstoffsäure eingegossen. Man rührt zwei Stunden bei Raumtemperatur und extrahiert mit Diethylether. Die Etherphase wird mit Natriumthiosulfatlösung und Wasser gewaschen, über Natriumsulfat getrocknet und im Vakuum eingedampft. Der ölige Rückstand wird an einer Silicagelsäule (Kromasil 100/10 µm) mit einem Hexan-*tert*-Butylmethylether-Gemisch (4:1) chromatographiert. Es werden 540 mg *(R,S)*-5-(3-Ethoxy-4-methoxyphenyl)-5-methyl-dihydro-2-furanon als öliges Produkt eluiert.

**Beispiel 8**

**5-(3-Cyclopentyloxy-4-methoxyphenyl)-5-ethyl-dihydro-2-furanon**

**A.** 100 ml einer 0.1 M Lösung von Samarium(II)-iodid in Tetrahydrofuran werden unter striktem Sauerstoffauschluß mit der Lösung von 1.0 g 3-Cyclopentyloxy-4-methoxypropiophenon, 500 mg Acrylsäureethylester und 350 mg *tert*-Butanol in 10 ml Tetrahydrofuran versetzt. Die Reaktionslösung wird gut durchmischt, 5 Stunden bei Raumtemperatur stehen gelassen und anschließend in 100 ml 20proz. Chlorwasserstoffsäure eingegossen. Man rührt zwei Stunden bei Raumtemperatur und extrahiert mit Diethylether. Die Etherphase wird mit Natriumthiosulfatlösung und Wasser gewaschen, über Natriumsulfat getrocknet und im Vakuum eingedampft. Der Rückstand, ein gelbes Öl, wird an einer Silicagelsäule (Kromasil 100/10 µm) mit einem Hexan-*tert*-Butylmethylether-Gemisch (4:1) chromatographiert. Es werden 770 mg öliges *(R,S)*-5-(3-Cyclopentyloxy-4-methoxyphenyl)-5-ethyl-dihydro-2-furanon eluiert.

## Patentansprüche

1. Racemische oder enantiomerenreine Verbindungen der Formel I,

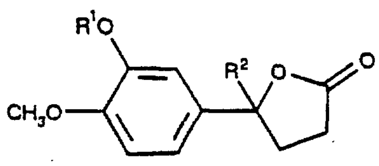

(I)

worin

$R^1$ einen Kohlenwasserstoffrest mit bis zu 8 C-Atomen und

$R^2$ $C_{1-4}$-Alkyl bedeutet.

2. *(R,S)*-5-(3,4-Dimethoxyphenyl)-5-methyl-dihydro-2-furanon
*(R,S)*-5-(3-Ethoxy-4-methoxyphenyl)-5-methyl-dihydro-2-furanon
*(R,S)*-5-(4-Methoxy-3-propoxyphenyl)-5-methyl-dihydro-2-furanon
*(R)*-(+)-5-(4-Methoxy-3-propoxyphenyl)-5-methyl-dihydro-2-furanon
*(S)*-(-)-5-(4-Methoxy-3-propoxyphenyl)-5-methyl-dihydro-2-furanon
*(R,S)*-5-(3-Isobutyloxy-4-methoxyphenyl)-5-methyl-dihydro-2-furanon
*(R)*-(+)-5-(3-Isobutyloxy-4-methoxyphenyl)-5-methyl-dihydro-2-furanon
*(S)*-(-)-5-(3-Isobutyloxy-4-methoxyphenyl)-5-methyl-dihydro-2-furanon
*(R,S)*-5-(3-Cyclopropylmethyloxy-4-methoxyphenyl)-5-methyl-dihydro-2-furanon
*(R)*-(+)-5-(3-Cyclopropylmethyloxy-4-methoxyphenyl)-5-methyl-dihydro-2-furanon
*(S)*-(-)-5-(3-Cyclopropylmethyloxy-4-methoxyphenyl)-5-methyl-dihydro-2-furanon
*(R,S)*-5-(3-Cyclobutyloxy-4-methoxyphenyl)-5-methyl-dihydro-2-furanon
*(R)*-(+)-5-(3-Cyclobutyloxy-4-methoxyphenyl)-5-methyl-dihydro-2-furanon
*(S)*-(-)-5-(3-Cyclobutyloxy-4-methoxyphenyl)-5-methyl-dihydro-2-furanon
*(R,S)*-5-(3-Cyclopentyloxy-4-methoxyphenyl)-5-methyl-dihydro-2-furanon
*(R)*-(+)-5-(3-Cyclopentyloxy-4-methoxyphenyl)-5-methyl-dihydro-2-furanon
*(S)*-(-)-5-(3-Cyclopentyloxy-4-methoxyphenyl)-5-methyl-dihydro-2-furanon
*(R,S)*-5-(3-Cyclopentyloxy-4-methoxyphenyl)-5-ethyl-dihydro-2-furanon nach Anspruch 1.

3. Arzneimittel enthaltend eine Verbindung nach Anspruch 1 oder 2 und einen oder mehrere übliche Träger- oder Hilfsstoffe.

4. Verwendung der Verbindungen nach Anspruch 1 oder 2 zur Herstellung eines Arzneimittels zur Behandlung von

Krankheiten, die durch die Aktivierung des Tumomekrosefaktors vermittelt werden.

5. Verwendung der Verbindungen nach Anspruch 1 oder 2 zur Herstellung eines Arzneimittels zur Behandlung Multipler Sklerose.

6. Verfahren zur Herstellung der Verbindungen nach Anspruch 1 **dadurch gekennzeichnet, daß** man eine Verbindung der Formel II,

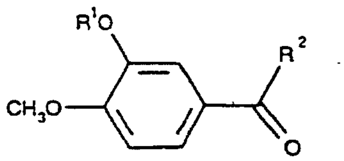

(II)

worin $R^1$ und $R^2$ die obige Bedeutung haben, mit einem Acrylsäureester in Gegenwart von Samarium(II)-iodid und einem Protonendonator umsetzt und gewünschtenfalls anschließend die Isomeren trennt.

7. Eine Verbindung nach Anspruch 1, **dadurch gekennzeichnet, daß** der Kohlenwasserstoffrest $R^1$ Alkyl, Alkenyl, Alkinyl, Cycloalkyl oder Cycloalkylalkyl bedeutet.

**Claims**

1. Racemic or enantiomer-pure compounds of formula I,

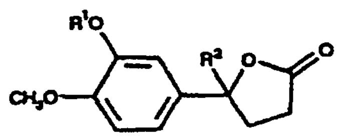

(I)

in which

$R^1$ means a hydrocarbon radical with up to 8 C atoms, and

$R^2$ means $C_{1-4}$-alkyl.

2. (R,S)-5-(3,4-Dimethoxyphenyl)-5-methyl-dihydro-2-furanone,
(R,S)-5-(3-ethoxy-4-methoxyphenyl)-5-methyl-dihydro-2-furanone,
(R,S)-5-(4-methoxy-3-propoxyphenyl)-5-methyl-dihydro-2-furanone,
(R)-(+)-5-(4-methoxy-3-propoxyphenyl)-5-methyl-dihydro-2-furanone,
(S)-(-)-5-(4-methoxy-3-propoxyphenyl)-5-methyl-dihydro-2-furanone,
(R,S)-5-(3-isobutyloxy-4-methoxyphenyl)-5-methyldihydro-2-furanone,
(R)-(+)-5-(3-isobutyloxy-4-methoxyphenyl)-5-methyldihydro-2-furanone,
(S)-(-)-5-(3-isobutyloxy-4-methoxyphenyl)-5-methyldihydro-2-furanone,
(R,S)-5-(3-cyclopropylmethyloxy-4-methoxyphenyl)-5-methyl-dihydro-2-furanone,
(R)-(+)-5-(3-cyclopropylmethyloxy-4-methoxyphenyl)-5-methyl-dihydro-2-furanone,
(S)-(-)-5-(3-cyclopropylmethyloxy-4-methoxyphenyl)-5-methyl-dihydro-2-furanone,
(R,S)-5-(3-cyclobutyloxy-4-methoxyphenyl)-5-methyldihydro-2-furanone,
(R)-(+)-5-(3-cyclobutyloxy-4-methoxyphenyl)-5-methyldihydro-2-furanone,
(S)-(-)-5-(3-cyclobutyloxy-4-methoxyphenyl)-5-methyldihydro-2-furanone,
(R,S)-5-(3-cyclopentyloxy-4-methoxyphenyl)-5-methyldihydro-2-furanone,
(R)-(+)-5-(3-cyclopentyloxy-4-methoxyphenyl)-5-methyldihydro-2-furanone,
(S)-(-)-5-(3-cyclopentyloxy-4-methoxyphenyl)-5-methyldihydro-2-furanone,
(R,S)-5-(3-cyclopentyloxy-4-methoxyphenyl)-5-ethyldihydro-2-furanone,

according to Claim 1

**3.** Pharmaceutical agents which comprise a compound according to Claim 1 or 2 and one or more customary vehicles or adjuvants.

**4.** Use of the compounds according to Claim 1 or 2 for the production of a pharmaceutical agent for treating diseases mediated by activation of the tumour necrosis factor.

**5.** Use of the compounds according to Claim 1 or 2 for the production of a pharmaceutical agent for treating multiple sclerosis.

**6.** Process for the production of the compounds according to Claim 1, **characterized in that** a compound of formula II,

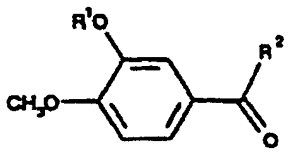

(II)

in which $R^1$ and $R^2$ have the above meaning is reacted with an acrylic acid ester in the presence of samarium(II) iodide and a proton donor, and optionally the isomers are separated.

**7.** A compound according to Claim 1, **characterized in that** the hydrocarbon radical $R^1$ is alkyl, alkenyl, alkinyl, cycloalkyl or cycloalkylalkyl.

**Revendications**

**1.** Composés racémiques ou énantiomériquement purs de formule I,

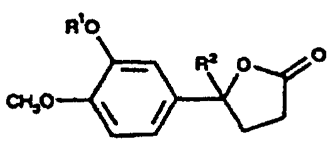

(I)

dans laquelle

$R^1$ représente un radical hydrocarboné ayant jusqu'à 8 atomes de carbone, et
$R^2$ représente un alkyle en $C_{1-4}$.

**2.** (R,S)-5-(3,4-Diméthoxyphényl)-5-méthyl-dihydro-2-furanone,
(R,S)-5-(3-éthoxy-4-méthoxyphényl)-5-méthyl-dihydro-2-furanone,
(R,S)-5-(4-méthoxy-3-propoxyphényl)-5-méthyl-dihydro-2-furanone,
(R)-(+)-5-(4-méthoxy-3-propoxyphényl)-5-méthyl-dihydro-2-furanone,
(S)-(-)-5-(4-méthoxy-3-propoxyphényl)-5-méthyl-dihydro-2-furanone,
(R,S)-5-(3-isobutyloxy-4-méthoxyphényl)-5-méthyldihydro-2-furanone,
(R)-(+)-5-(3-isobutyloxy-4-méthoxyphényl)-5-méthyldihydro-2-furanone,
(S)-(-)-5-(3-isobutyloxy-4-méthoxyphényl)-5-méthyldihydro-2-furanone,
(R,S)-5-(3-cyclopropylméthyloxy-4-méthoxyphényl)-5-méthyl-dihydro-2-furanone,
(R)-(+)-5-(3-cyclopropylméthyloxy-4-méthoxyphényl)-5-méthyl-dihydro-2-furanone,
(S)-(-)-5-(3-cyclopropylméthyloxy-4-méthoxyphényl)-5-méthyl-dihydro-2-furanone,
(R,S)-5-(3-cyclobutyloxy-4-méthoxyphényl)-5-méthyldihydro-2-furanone,
(R)-(+)-5-(3-cyclobutyloxy-4-méthoxyphényl)-5-méthyldihydro-2-furanone,
(S)-(-)-5-(3-cyclobutyloxy-4-méthoxyphényl)-5-méthyldihydro-2-furanone,

(R,S)-5-(3-cyclopentyloxy-4-méthoxyphényl)-5-méthyldihydro-2-furanone,
(R)-(+)-5-(3-cyclopentyloxy-4-méthoxyphényl)-5-méthyldihydro-2-furanone,
(S)-(-)-5-(3-cyclopentyloxy-4-méthoxyphényl)-5-méthyldihydro-2-furanone,
(R,S)-5-(3-cyclopentyloxy-4-méthoxyphényl)-5-éthyldihydro-2-furanone,
selon la revendication 1.

**3.** Médicaments comprenant un composé selon la revendication 1 ou 2 et un ou plusieurs supports ou auxiliaires habituels.

**4.** Utilisation des composés selon la revendication 1 ou 2 pour la production d'un médicament destiné au traitement de maladies dont la médiation est assurée par l'activation du facteur de nécrose tumorale.

**5.** Utilisation des composés selon la revendication 1 ou 2 pour la production d'un médicament destiné au traitement de la sclérose en plaques.

**6.** Procédé de production des composés selon la revendication 1, **caractérisé en ce qu'**un composé de formule II,

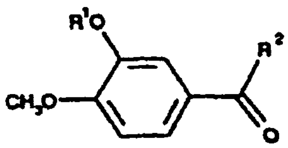

(II)

dans laquelle $R^1$ et $R^2$ présentent la signification ci-dessus, est mis à réagir avec un ester de l'acide acrylique en présence d'iodure de samarium(II) et d'un donneur de proton et les isomères sont ensuite séparés, si on le souhaite.

**7.** Composé selon la revendication 1, **caractérisé en ce que** le radical hydrocarboné $R^1$ représente un alkyle, un alcényle, un alcynyle, un cycloalkyle ou un cycloalkylalkyle.